(19) Europäisches Patentamt
European Patent Office
Office européen des brevets

(11) Publication number: **0 104 775**
**B1**

(12) # EUROPEAN PATENT SPECIFICATION

(45) Date of publication of patent specification: **20.08.86**

(51) Int. Cl.[4]: **C 07 F 9/38,** C 07 C 103/48, C 07 F 9/40

(21) Application number: **83304951.3**

(22) Date of filing: **26.08.83**

(54) **Production of N-phosphonomethylglycine.**

(30) Priority: **30.08.82 US 412624**
**22.02.83 US 468223**

(43) Date of publication of application:
**04.04.84 Bulletin 84/14**

(45) Publication of the grant of the patent:
**20.08.86 Bulletin 86/34**

(84) Designated Contracting States:
**AT BE CH DE FR GB IT LI NL SE**

(56) References cited:
**US-A-3 923 877**
**US-A-3 988 142**
**US-A-4 053 505**

(73) Proprietor: **STAUFFER CHEMICAL COMPANY**
**Westport Connecticut 06881 (US)**

(72) Inventor: **Felix, Raymond Anthony**
**1662 Shasta Street**
**Richmond California 94804 (US)**

(74) Representative: **Smith, Sydney et al**
**Elkington and Fife High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the production of N-phosphonomethylglycine.

Certain salts of N-phosphonomethylglycine are particularly effective as post-emergence herbicides. The commercial herbicide is sold as a formulation containing the isopropylamine salt of N-phosphonomethylglycine.

N-phosphonomethylglycine may be obtained by a number of methods. One such method, as described in US—A—3,160,632 is to react N-phosphonomethylglycine (glycine methylene phosphonic acid) with mercuric chloride in water at reflux temperature and subsequently to separate the reaction products. Other methods are phosphonomethylation of glycine and the reaction of ethyl glycinate with formaldehyde and diethylphosphite. The latter method is described in US—A—3,799,758. In addition, there is a series of patents relating to the production of N-phosphonomethylglycine, including US—A—3,868,407; US—A—4,197,254, and US—A—4,199,354.

Also, US—A—3,923,877 teaches the reaction of 1,3,5-tricyanomethylhexahydro-1,3,5-triazine with excess disubstituted phosphite to form $(RO)_2P(O)CH_2NHCH_2CN$ wherein R represents hydrocarbyl or substituted hydrocarbyl) which is hydrolyzed to yield N-phosphonomethylglycine.

Moreover, US—A—4,053,505 relates to the production of N-phosphonomethylglycine by reacting a 1,3,5,-trihydrocarbyloxycarbonyl-methylhexahydrotriazine with a di-monovalent hydrocarbon phosphite to form an ester, which is hydrolyzed to yield the desired product.

Furthermore, US—A—3,988,142, which relates to a method of increasing carbohydrate deposition in plants using N-phosphonomethylglycine and certain derivatives thereof, refers, *inter alia,* to methyl N-acetyl-N-(diethoxyphosphinylmethyl)glycinate.

Because of the commercial importance of certain salts of N-phosphonomethylglycine as herbicides, improved methods of preparing N-phosphonomethylglycine as well as the salts are valuable.

This invention relates to a process for the production of N-phosphonomethylglycine characterized in that it comprises:

(a) reacting a 1,3,5-trihydroxycarbonylmethylhexahydro-1,3,5-triazine derivative of the formula:

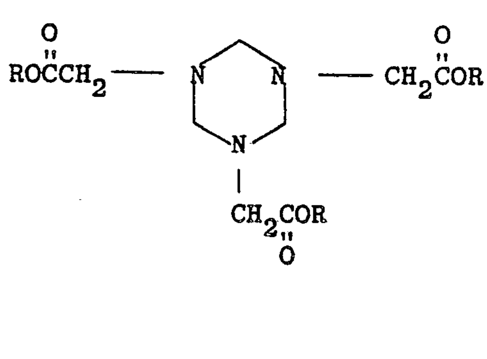

wherein R represents an aliphatic group having from 1 to 10 carbon atoms, an aromatic group having from 6 to 20 carbon atoms or a N—, S—, or O— containing heterocyclic group having from 5 to 19 carbon atoms and having an unsaturated ring structure, preferably $C_1$—$C_4$ alkyl, most preferably methyl or ethyl; with an acyl halide of the formula:

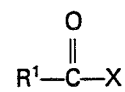

wherein X represents chlorine, bromine or iodine, preferably chlorine; and $R^1$ has the same definition as R; to form the N-oxycarbonylmethyl-N-halomethylamide derivative of the acyl halide of the formula:

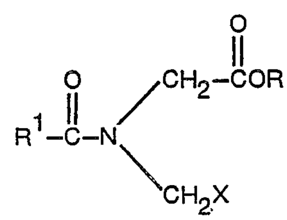

(b) reacting the resulting amide with a phosphite of the formula:

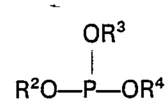

wherein $R^2$ and $R^3$ both have the same definition as R, preferably $R^2$ and $R^3$ are $C_1$—$C_6$ alkyl, more preferably $C_1$—$C_4$ alkyl; and $R^4$ represents an aliphatic group having from 1 to 10 carbon atoms, preferably $C_1$—$C_6$ alkyl, more preferably $C_1$—$C_4$ alkyl, or an alkali metal (M), preferably sodium or potassium; to form a phosphonate compound of the formula:

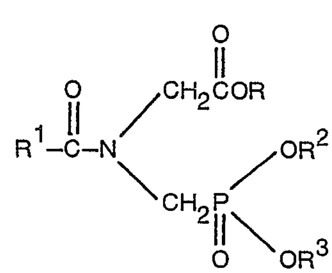

($R^4X$ being the other product of the reaction) and

(c) hydrolyzing the resulting phosphonate.

(c) 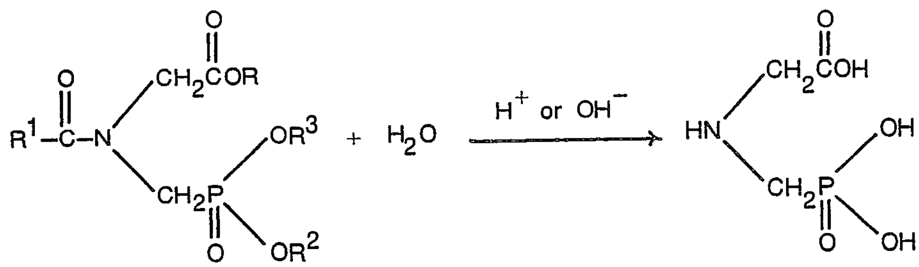


wherein R, $R^1$, $R^2$ and $R^3$ are as defined above and $H^+$ is the proton of a strong acid such as hydrochloric, hydrobromic, hydriodic, nitric, sulfuric, phosphonic or chloroacetic acid. Preferably $H^+$ is the proton of hydrochloric or hydrobromic acid and $OH^-$ is the anion of a strong base such as sodium hydroxide or potassium hydroxide, preferably in an aqueous, aqueous-alcoholic or alcoholic solution. Preferably, the hydrolysis is run in the presence of a strong acid.

In the above reaction scheme, the groups R and $R^1$ are not directly involved in reaction step (a) between the 1,3,5-trihydroxycarbonylmethylhexahydro-1,3,5-triazine derivative and the acyl halide. Groups R, $R^1$, $R^2$ or $R^3$ are not directly involved in reaction step (b) between the N-alkoxycarbonylmethyl-N-chloromethylamide reaction product of step (a) and the phosphite. Groups R, $R^1$, $R^2$ and $R^3$ are removed in reaction step (c) when the phosphonate reaction product of reaction step (b) is subjected to hydrolysis. Therefore, the nature of groups R, $R^1$, $R^2$ and $R^3$ is not critical, although groups which would interfere with reaction steps (a) and (b) are to be avoided.

The group "$C_1$—$C_4$ alkyl" encompasses methyl, ethyl, n-propyl, isopropyl, n-butyl, isobutyl, sec-butyl, and tert-butyl. The group "$C_1$—$C_6$ alkyl" encompasses the same radicals as $C_1$—$C_4$ alkyl plus the 6 pentyls and the 16 hexyls.

The term "aliphatic group" covers organic groups characterized by being derived from (1) an acyclic (open-chain structure) of the paraffin, olefin and acetylene hydrocarbon series or (2) alicyclic compounds. The aliphatic group has from 1 to 10 carbon atoms.

The term "aromatic group" is used in a broad sense to distinguish from the aliphatic group and includes a group derived from compounds having 6 to 20 carbon atoms and characterized by the presence of at least one benzene ring, including monocyclic, bicyclic and polycyclic hydrocarbons. The term "heterocyclic compounds" includes compounds having from 5 to 19 carbon atoms which are similar in structure and are characterized by having an unsaturated ring structure containing at least one atom other than carbon selected from nitrogen, sulfur and oxygen.

Reaction step (a) is preferably run at a temperature from 0° to 150°C, more preferably 40° to 110°C and most preferably between 75° to 85°C. This reaction step can be run at atmospheric, subatmospheric or super-atmospheric pressure, preferably at atmospheric pressure. Preferably the reaction is run in a solvent for the acyl halide, such as ethylene dichloride, methylene chloride, tetrahydrofuran or toluene.

Three moles of the acyl halide are needed to react with one mole of the 1,3,5-trihydroxycarbonylmethylhexahydro-1,3,5-triazine derivative. An excess of acyl halide can be used to ensure complete reaction with the triazine. A large excess of the acyl halide can serve as a solvent in this reaction step. The solvent or any excess acyl halide can be removed to isolate the N-oxycarbonylmethyl-N-chloromethylamide derivative of the acyl halide in high yields. However, this amide quickly degrades by hydrolysis and should be kept in an inert atmosphere if isolated. Most preferably no excess acyl halide is used.

In reaction step (b), most preferably about equal mole amounts of N-oxycarbonylmethyl-N-halomethylamide derivative of the acyl halide and the phosphite are reacted. Less preferably, up to 2 mole excess can be used and least preferably up to a 10 mole excess can be used.

The reaction is exothermic and can be run at a temperature between 0 to 150°C, more preferably between 40° to 100°C, most preferably between 75° to 85°C.

No solvent is needed for the reaction, however, any inert solvent can be used, preferably the solvent having a boiling point between 40° to 100°C. Examples of such solvents are ethylene chloride, methylene chloride, tetrahydrofuran and toluene. The use of an inert solvent helps dissipate the heat of reaction. Any solvent used in this reaction step will be removed after completion of reaction step (c), so preferably it is one that can be removed by evaporation.

Alkali metal phosphites having the formula

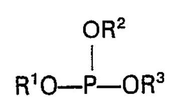

wherein $R^1$ and $R^2$ are as defined and $R^3$ is an alkali metal are reacted with N-cyanomethyl-N-halomethylamide under an inert atmosphere such as nitrogen. The alkali phosphite can be prepared by reacting an alkali metal alkoxide, alkali metal hydride or alkali metal with an equal mole amount of a disubstituted phosphite of the formula

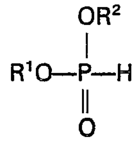

wherein $R^1$ and $R^2$ are as defined. This reaction is run in an inert atmosphere such as nitrogen.

Alkali metal phosphites of the formula

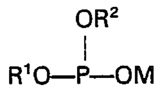

wherein $R^1$, $R^2$ and M are as defined can, because of tautomerism, have the following additional structural formula

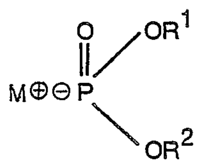

wherein $R^1$ and $R^2$ are as defined and M is an alkali metal.

In reaction step (c), a mole of the phosphonate reaction product from reaction step (b) is hydrolyzed with 5 moles of water. The hydrolysis is run in the presence of a strong acid or base as defined above. Preferably the hydrolysis is acid-catalyzed, preferably with an inorganic acid, and most preferably with hydrochloric or hydrobromic acid. The hydrolysis yields the desired N-phosphonomethylglycine. Preferably at least 2 moles of the acid are used. More, preferably, a large excess over the 2 mole amount is used. The preferred hydrochloric or hydrobromic acid can be used in concentrated or aqueous form.

This last reaction step is run at a temperature between 0 to 200°C, preferably between 50° to 125°C and most preferably between 100° to 125°C.

Atmospheric, sub-atmospheric or super-atmospheric pressure can be used. Preferably atmospheric pressure is used during the hydrolysis.

The solid N-phosphonomethylglycine can be recovered by conventional techniques in reaction step (c). Volatile liquid products such as alcohols (methanol), aliphatic halides (methyl chloride), acids (acetic acid), water, and excess acid and be removed by standard stripping techniques. The desired N-phosphonomethylglycine is recovered in high purity by titurating it in isopropyl alcohol and removing it by filtration.

The process of this invention can be better understood by reference to the following specific examples.

Example 1

Preparation of N-ethoxycarbonylmethyl-N-chloromethylacetamide

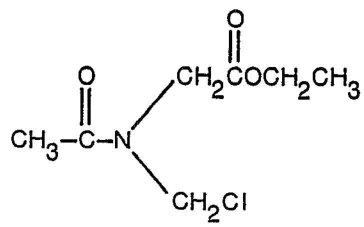

1,3,5-Tri-ethoxycarbonylmethylhexahydro-1,3,5-triazine (5.76 g, 0.0167 mole) was dissolved in 150 ml of 1,2-dichloroethane in a round-bottom flask. Acetyl chloride (5.4 ml, 0.074 mole) was added dropwise. The reaction mixture was refluxed 15 minutes, then stripped under reduced pressure to yield N-ethoxycarbonylmethyl-N-chloromethylacetamide. The structure was confirmed by proton nuclear magnetic resonance.

Example 2

Preparation of O,O-dimethyl-N-ethoxycarbonyl-methyl-N-acetylaminomethyl phosphonate

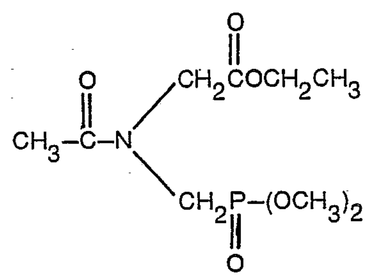

The amide compound prepared in Example 1 was diluted with 10—12 ml of toluene. Trimethyl phosphite (6.7 g, 0.0515 mole) was added, and the mixture was refluxed 15 minutes, then stripped under reduced pressure to yield the desired product. The structure was confirmed by proton nuclear magnetic resonance.

Example 3

Preparation of N-phosphonomethylglycine

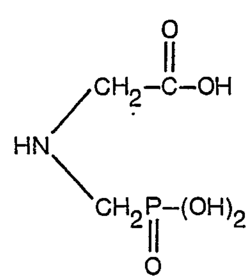

The phosphonate reaction product of Example 2 was combined with 30 ml (0.36 mole) of concentrated hydrochloric acid, refluxed 3 hours, and stripped under reduced pressure. The product was titurated in 50 ml of isopropyl alcohol and filtered to yield 5.6 g of the desired product. The structure was confirmed by proton nuclear magnetic resonance, $13_C$ nuclear magnetic resonance, infrared, and liquid chromatography.

Example 4
Preparation of O,O-diethyl-N-ethoxycarbonylmethyl-N-acetylaminomethyl phosphonate

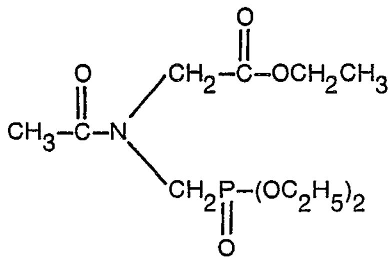

3.9 grams (0.035 ml) of potassium-butoxide were slurried in a round bottom flask with 25 ml tetrahydrofuran (dried over molecular sieves) and the slurry was cooled in a water bath. 4.5 ml (0.035 mole) of diethyl phosphite were added dropwise over 5 minutes under nitrogen. This mixture was then cooled in an ice bath and 6.77 g (0.035 mole) of N-ethoxycarbonylmethyl-N-chloromethylacetamide diluted with 25 ml of tetrahydrofuran were added dropwise over 15 minutes. The mixture was allowed to warm to ambient temperature and stirred 3 hours before it was filtered through "Dicalite" (Trade Mark) and stripped to yield 9.6 g of an amber oil. Structure was confirmed by ir, nmr, ms, and C—13 nmr.

Example 5
Preparation of N-phosphonomethylglycine

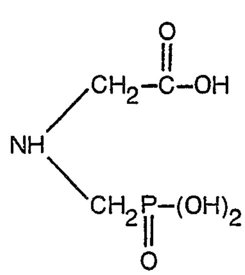

6.6 g (0.022 mole) of the compound obtained from Example 4 were combined with 30 ml (0.363 mole) of concentrated HCll and refluxed 3 hours then stripped to yield 4.7 g of the desired product, a brown semi-solid. Structure was confirmed by $H^1$, nmr, C—13 nmr and lc techniques.

**Claims**

1. A process for the production of N-phosphonomethylglycine characterized in that it comprises:

(a) reacting a 1,3,5-trihydroxycarbonylmethylhexahydro-1,3,5-triazine of the formula:

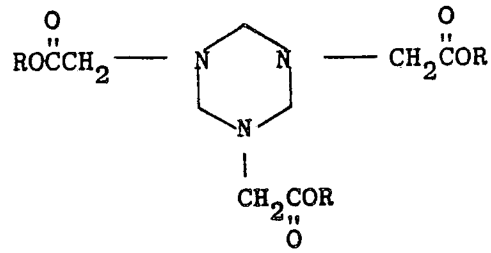

wherein R represents an aliphatic group having from 1 to 10 carbon atoms, an aromatic group having from 6 to 20 carbon atoms or a N—, S— or O— containing heterocyclic group having from 5 to 19 carbon atoms and having an unsaturated ring structure; with an acyl halide of the formula:

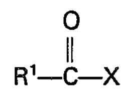

wherein X represents chlorine, bromine or iodine; and $R^1$ has the same definition as R; to form the N-oxycarbonylmethyl-N-halomethylamide derivative of the acyl halide of the formula:

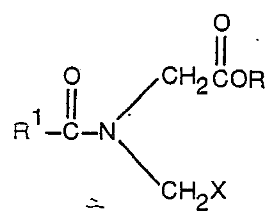

(b) reacting the resulting amide with a phosphite of the formula:

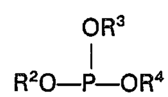

wherein $R^2$ and $R^3$ both have the same definition as R; and $R^4$ represents an aliphatic group having from 1 to 10 carbon atoms or an alkali metal; to form a phosphonate compound of formula:

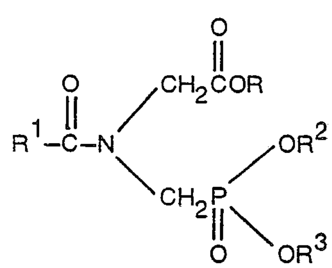

and

(c) hydrolyzing the resulting phosphonate.

2. A process as claimed in claim 1 wherein R and $R^1$ represent $C_1$—$C_4$ alkyl and X represents chlorine; or R and $R^1$ represent $C_1$—$C_4$ alkyl, $R^2$ represents $C_1$—$C_6$ alkyl, $R^3$ represents $C_1$—$C_6$ alkyl, $R^4$ represents $C_1$—$C_6$ alkyl; sodium or potassium and X represents chlorine; or R and $R^1$ represent $C_1$ or $C_2$ alkyl, $R^2$ represents $C_1$—$C_4$ alkyl, $R^3$ represents $C_1$—$C_4$ alkyl, $R^4$ represents $C_1$—$C_4$ alkyl, sodium or potassium and X represents chlorine; or R, $R^1$, $R^2$. $R^3$ and $R^4$ represent $C_1$ or $C_2$ alkyl and X represents chlorine; or R represents ethyl, $R^1$, $R^2$ and $R^3$ represent methyl and X represents chlorine.

3. A process as claimed in claim 1 or claim 2 wherein, for (a), the temperature is from 0 to 150°C.

4. A process as claimed in any of claims 1 to 3 wherein, for (c), an acid catalyst is used.

5. A process as claimed in claim 4 wherein the acid catalyst is hydrochloric or hydrobromic acid.

6. A compound characterised by the formula:

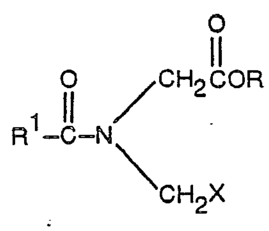

wherein R and $R^1$ represent $C_1$—$C_4$ alkyl and X represents chlorine, bromine or iodine.

7. A compound as claimed in claim 6 wherein R represents ethyl, $R^1$ represents methyl and X represents chlorine.

**Patentansprüche**

1. Verfahren zur Herstellung von N-Phosphonomethylglycin dadurch gekennzeichnet, daß

(a) ein 1,3,5-Tricarboxymethylhexahydro-1,3,5-triazinderivat mit der Formel

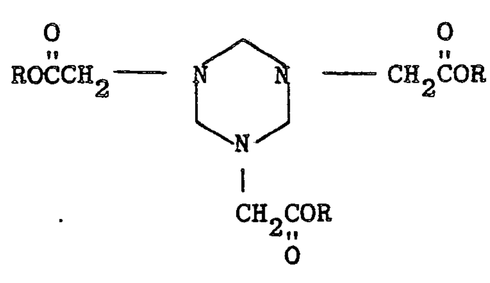

in der R ein aliphatischer Rest mit 1 bis 10 Kohlenstoffatomen, ein aromatischer Rest mit 6 bis 20 Kohlenstoffatomen oder ein ungesättigter, 5 bis 19 Kohlenstoffatome aufweisender Heterocyclus mit Stickstoff-, Schwefel- oder Sauerstoffheteroatomen ist, mit einem Säurehalogenid der Formel

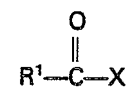

in der X Chlor, Brom oder Jod ist une $R^1$ die gleiche Bedeutung wie R hat, umgesetzt wird, wobei ein N-Carboxymethyl-N-halomethylamidderivat des Säurehalogenids mit der Formel

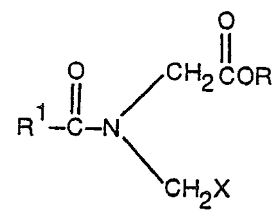

gebildet wird,

(b) das erhaltene Amid mit einem Phosphit mit der Formel

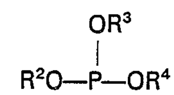

in der $R^2$ und $R^3$ die gleiche Bedeutung wie R haben und $R^4$ ein aliphatischer Rest mit 1 bis 10 Kohlenstoffatomen oder ein Alkalimetall ist, umgesetzt wird, wobei ein Phosphonat mit der Formel

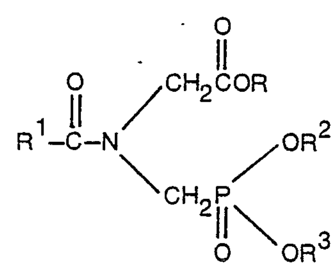

gebildet wird, und

(c) das erhaltene Phosphonat hydrolisiert wird.

2. Verfahren nach Anspruch 1, dadurch gekennzeichnet, daß R und $R^1$ $C_1$—$C_4$-Alkyl sind und X Chlor ist, oder R und $R^1$ $C_1$—$C_4$-Alkyl sind und $R^2$ $C_1$—$C_6$-Alkyl, $R^3$ $C_1$—$C_6$-Alkyl und $R^4$ $C_1$—$C_6$-Alkyl, Natrium oder Kalium sind und X Chlor ist, oder R und $R^1$ $C_1$-oder $C_2$ Alkyl sind, $R^2$ $C_1$—$C_4$-Alkyl, $R^3$ $C_1$—$C_4$-Alkyl, $R^4$ . $C_1$—$C_4$-Alkyl, Natrium oder Kalium sind und X Chlor ist, oder R, $R^1$, $R^2$, $R^3$ und $R^4$ $C_1$-oder $C_2$ Alkyl sind und X Chlor ist, oder R Ethyl ist und $R^1$, $R^2$ und $R^3$ Methyl sind und X Chlor ist.

3. Verfahren nach Anspruch 1 oder 2, dadurch gekennzeichnet, daß bei Schritt (a) die Temperatur 0 bis 150°C beträgt.

4. Verfahren nach einem der Ansprüche 1 bis 3, dadurch gekennzeichnet, daß bei Schritt (c) ein saurer Katalysator verwendent wird.

5. Verfahren nach Anspruch 4, dadurch gekennzeichnet, daß der saure Katalysator Chlorwasserstoffsäure oder Bromwasserstoffsäure ist.

6. Stoff, gekennzeichnet durch die Formel

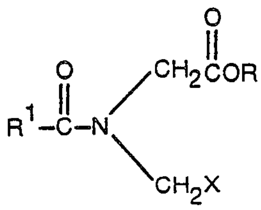

in der R und $R^1$ $C_1$—$C_4$-Alkyl sind und X Chlor, Brom oder Jod ist.

7. Stoff nach Anspruch 6, dadurch gekennzeichnet, daß R Ethyl ist, $R^1$ Methyl ist und X Chlor ist.

**Revendications**

1. Un procédé de préparation de la N-phosphonométhylglycine, caractérisé en ce qu'il comprend:

(a) la réaction d'un dérivé de la 1,3,5-trihydroxycarbonylméthylhexahydro-1,3,5-triazine de formule:

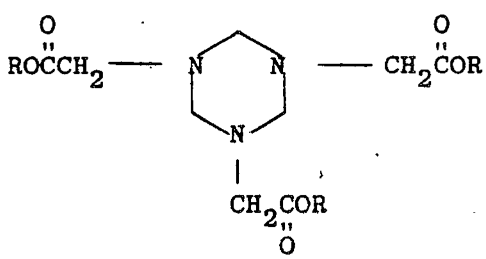

dans laquelle:

R répresente un group aliphatique comprenant de 1 à 10 atomes de carbone, un groupe aromatique comprenant de 6 à 20 atomes de carbone ou un groupe hétérocyclique contenant de l'azote, du soufre ou de l'oxygène, comprenant de 5 à 19 atomes de carbone et une structure cyclique insaturée, de préférence un groupe alkyle en $C_1$—$C_4$ et, de façon préférée, un groupe méthyle ou étyle,

avec un halogénure d'acyle de formule:

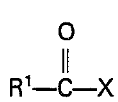

dans laquelle:

X représente du chlore, du brome ou de l'iode, de préférence du chlore; et

$R^1$ a la même définition que R;

pour former le N-oxycarbonylméthyle-N-halométhylamide dérivé de l'halogénure d'acyle de formule:

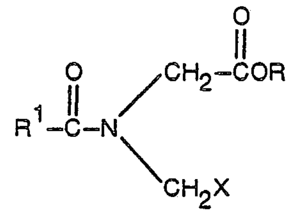

(b) la réaction de l'amide résultante avec un phosphite de formule:

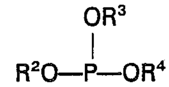

dans laquelle:

$R^2$ et $R^3$ ont, la même définition que R, de préférence $R^2$ et $R^3$ représentent des groupes alkyles en $C_1$—$C_6$, plus préférablement des groupes alkyles en $C_1$—$C_4$; et

$R^4$ représente un groupe aliphatique comportant de 1 à 10 atomes de carbone, de préférence un groupe alkyle en $C_1$—$C_6$ et, de manière préférée. un groupe alkyle en $C_1$—$C_4$ ou un métal alcalin (M), de préférence du sodium ou du potassium;

pour former un phosphonate de formule:

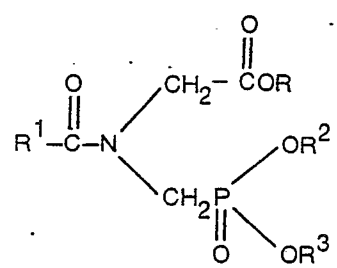

($R^4X$ étant produit de la réaction);

et

(c) l'hydrolyse du phosphonate résultant.

2. Un procédé selon la revendication 1, dans lequel:

R et $R^1$ représentent un groupe alkyle en $C_1$—$C_4$; et

X représente du chlore;

ou

R et $R^1$ représentent un group alkyle en $C_1$—$C_4$;

$R^2$ représente un groupe alkyle en $C_1$—$C_6$;

$R^3$ représente un groupe alkyle en $C_1$—$C_6$;

$R^4$ représente un groupe alkyle en $C_1$—$C_6$, du sodium ou du potassium; et

X représente du chlore;

ou

R et $R^1$ représentent un alkyle en $C_1$ ou $C_2$;

$R^2$ représente un groupe alkyle en $C_1$—$C_4$;

$R^3$ représente un groupe alkyle en $C_1$—$C_4$;

$R^4$ représente un groupe alkyle en $C_1$—$C_4$, du sodium ou du potassium et

X représente du chlore;
ou
R, $R^1$, $R^2$, $R^3$ et $R^4$ représente un alkyle en $C_1$ ou $C_2$; et
X représente du chlore;
ou
R représente un groupe éthyle;
$R^1$, $R^2$ et $R^3$ représentent un groupe méthyle; et
X représente du chlore.

3. Un procédé selon la revendication 1 ou 2, dans lequel dans (a) la température est comprise entre 0° et 150°C.

4. Un procédé selon l'une quelconque des revendications 1 à 3, dans lequel dans (c) est utilisé un catalysateur acide.

5. Un procédé la revendication 4, dans lequel le catalyseur acide est l'acide chlorhydrique ou l'acide bromhydrique.

6. Un composé caractérisé par la formule:

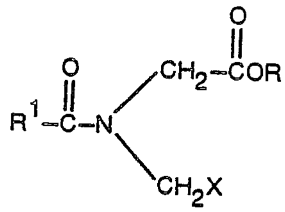

dans laquelle:
R et $R^1$ représentent un groupe alkyle en $C_1$—$C_4$; et
X représentent du chlore, du brome ou de l'iode.

7. Un composé selon la revendication 6, dans lequel:
R représente un groupe éthyle;
$R^1$ représente un groupe méthyle; et
X représente du chlore.